# EUROPÄISCHE PATENTSCHRIFT

(11) **EP 1 151 985 B1**
(45) Veröffentlichungstag und Bekanntmachung des Hinweises auf die Patenterteilung: **17.12.2003**
(21) Anmeldenummer: 01107090.1
(22) Anmeldetag: 22.03.2001
(51) Int. Cl.: C07C 37/08

(54) **Verfahren zur thermischen Nachbehandlung von Spaltprodukt aus der säurekatalysierten Spaltung von Cumolhydroperoxid**
Process for the thermal aftertreatment of the splitting product of the acid catalysed splitting of cumylhydroperoxide
Procédé pour le posttraitement thermique du produit de scission de la scission par catalyse acide de l'hydropéroxyde de cumène

(30) Priorität: 03.05.2000 DE 10021482
(43) Veröffentlichungstag der Anmeldung: 07.11.2001
(73) Patentinhaber: INEOS Phenol GmbH & Co. KG, 45966 Gladbeck (DE)
(72) Erfinder: Weber, Manfred, Dr., 45721 Haltern (DE); Tanger, Uwe, Dr., 44879 Bochum (DE); Sigg, Reinhard, Dr., 45772 Marl (DE); Schnurr, Otto, Dr., 2950 Putte-Kapellen (BE); Liefooghe, Hugo H.J.M., Dr., 2650 Edegem (BE)
(74) Vertreter: Sternagel, Fleischer, Godemeyer & Partner

(56) Entgegenhaltungen:
- EP-A- 0 069 806
- DE-A- 19 755 026

## Beschreibung

Die Erfindung betrifft ein verbessertes Verfahren zur selektiven, energiesparenden thermischen Nachbehandlung von Spaltprodukt aus der säurekatalysierten Spaltung von Cumolhydroperoxid (CHP) in Phenol und Aceton.

Das Verfahren der säurekatalysierten Spaltung von Cumolhydroperoxid in Phenol und Aceton ist seit langem von besonderer industrieller Bedeutung. Bei dieser Herstellung von Phenol aus Cumol nach dem Hock-Verfahren wird in einer ersten Reaktionsstufe, der so genannten Oxidation, Cumol zu Cumolhydroperoxid (CHP) oxidiert und das CHP anschließend in einer Vakuumdestillation, der so genannten Konzentrierung auf 65 bis 90 Gew.-% aufkonzentriert. In einer zweiten Reaktionsstufe, der so genannten Spaltung, wird das CHP durch Einwirken einer Säure, zumeist Schwefelsäure, in Phenol und Aceton gespalten. Dabei wird das bereits in der Oxidation gebildete Dimethylphenylcarbinol (DMPC) in einer Gleichgewichtsreaktion zum Teil in α-Methylstyrol (AMS) und Wasser gespalten, ein weiterer Teil des DMPCs reagiert mit CHP zu Dicumylperoxid (DCP), der Rest verbleibt in dem so genannten Spaltprodukt. Nach Neutralisation des Spaltproduktes wird dieses Produktgemisch destillativ aufgearbeitet.

Ein Teil des AMS bildet in der Spaltung Hochsieder (Dimere, Cumylphenole), die in der Destillation als Rückstand ausgeschleust werden. Das nach der Neutralisation noch vorliegende AMS wird in der Destillation zu Cumol hydriert und zur Oxidation zurückgeführt. In der Spaltung nicht umgesetztes DMPC gelangt als Hochsieder in den Rückstand, zum Teil setzt es sich in den heißen Phenolkolonnen weiter um zu AMS, aus dem wiederum hochsiedende Nebenkomponenten entstehen. Das DCP ist bei üblichen Spalttemperaturen (50 bis 70 °C) stabil. Es zersetzt sich thermisch in den heißen Phenolkolonnen, wobei nach unseren Erfahrungen vor allem o-Kresole gebildet werden. Unter Einfluss von Säure kann DCP bei Temperaturen oberhalb 80 °C dagegen in Phenol, Aceton und AMS gespalten werden. Es liegt daher nahe, unmittelbar nach der Spaltung das restliche DMPC und das in der Spaltung gebildete DCP vollständig umzusetzen, und zwar durch gezielte Erhöhung der Temperatur unter Einfluss der in der Spaltung als Katalysator verwendeten Säure. Dadurch wird DMPC fast vollständig in AMS und DCP vollständig in Phenol, Aceton und ebenfalls AMS umgewandelt.

Eine derartige thermische Nachbehandlung des Spaltproduktes wurde bereits in US 2 757 209 beschrieben, wobei Temperaturen über 100°C, speziell von 110 bis 120°C verwendet wurden. Ziel dieser thermischen Nachbehandlung war die vollständige Dehydratisierung des DMPC zu AMS. In US 4 358 618 wird dagegen eine thermische Nachbehandlung beschrieben, die zum Ziel hat, das in der Spaltung gebildete DCP vollständig in Phenol, Aceton und AMS zu überführen, wobei Temperaturen von 120 und 150 °C verwendet werden. In US 5 254 751 wird eine thermische Nachbehandlung mit gleicher Zielsetzung wie in US 4 358 618 beschrieben, wobei hier Temperaturen von 80 bis 110 °C verwendet werden. In DE 197 55 026 A1 schließlich wird die Nachbehandlung in einem Temperaturbereich von über 150 °C durchgeführt. Demnach gibt es in den bisherigen Beschreibungen drastische Unterschiede hinsichtlich optimaler Temperaturbereiche für die thermische Nachbehandlung von Spaltprodukt aus der Phenolherstellung.

In all diesen bisher beschriebenen Verfahren wird das Spaltprodukt für die thermische Nachbehandlung in Wärmeübertragern mit Dampf zunächst aufgeheizt und nach ausreichender Reaktionszeit in Wärmeübertragern mit Wasser wieder abgekühlt. Je nach gewählter Temperatur für die thermische Nachbehandlung ergeben sich dadurch spezifische Dampfverbräuche um 0,2 Tonnen Dampf je Tonne Phenol. Es wurde von uns festgestellt, dass generell bei Temperaturen über 100 °C, vor allem bei Temperaturen über 120 °C ein verstärktes Ablagern von hochsiedenden Nebenprodukten in den Wärmeübertragern (Fouling) der thermischen Nachbehandlung, verbunden mit einem drastischen Rückgang des Wärmeübergangs auftritt. Insbesondere in den Apparaten zur Aufheizung des Produktes mit Dampf kommt es an den heißen Wärmeübertragungsflächen auf der Produktseite zur Ausbildung organischer Ablagerungen, so dass diese Apparate in relativ kurzen Zeitabständen von wenigen Wochen gereinigt werden müssen. Mit zunehmender Temperatur steigt dieses Fouling weiter an.

Damit stellt sich die Aufgabe, ein Verfahren zur thermischen Nachbehandlung von Spaltprodukt aus der Cumolhydroperoxidspaltung bereitzustellen, das sich neben hoher Selektivität auch durch niedrige Energiekosten sowie durch eine hohe Verfügbarkeit durch Vermeidung von Fouling auszeichnet.

Überraschenderweise wurde gefunden, dass ein Verfahren zur thermischen Nachbehandlung von Spaltprodukt aus der säurekatalysierten Spaltung von Cumolhydroperoxid in Phenol und Aceton, wobei das thermisch zu behandelnde Spaltprodukt in einem Reaktor erwärmt wird, welches dadurch gekennzeichnet ist, dass zum Erwärmen des thermisch zu behandelnden Spaltproduktes im Reaktor die Reaktionswärme zumindest einer in diesem Reaktor ablaufenden exothermen Reaktion genutzt wird, eine hohe Selektivität der Nachbehandlung bei gleichzeitiger Senkung der Energiekosten sowie höhere Standzeit der Wärmeübertrager durch Vermeiden von Fouling, erreicht.

Gegenstand der vorliegenden Erfindung ist deshalb ein Verfahren gemäß Anspruch 1 zur thermischen Nachbehandlung von Spaltprodukt aus der säurekatalysierten Spaltung von Cumolhydroperoxid in Phenol und Aceton, wobei das thermisch zu behandelnde Spaltprodukt in einem Reaktor erwärmt wird, welches dadurch gekennzeichnet ist, dass zum Erwärmen des thermisch zu behandelnden Spaltproduktes im Reaktor die Reaktionswärme zumindest einer in diesem Reaktor ablaufenden exothermen Reaktion genutzt wird.

Gemäß einer bevorzugten Ausführungsform der vorliegenden Erfindung wird das Verfahren zur Herstellung von Phenol und Aceton durch säurekatalysierte Spaltung von Cumolhydroperoxid in einem Reaktor durchgeführt, der zumindest zwei Bereiche aufweist, von denen zumindest ein Bereich mit einer Vorrichtung zum Abführen von Wärme ausgerüstet ist und zumindest ein weiterer Bereich Strömungsrohrcharakteristik aufweist.

Diese bevorzugte Ausführungsform hat den Vorteil, dass die eigentliche CHP-Spaltung und die thermische Nachbehandlung in einem Reaktor kombiniert durchgeführt werden können.

Das erfindungsgemäße Verfahren hat den Vorteil, dass gegenüber herkömmlichen Verfahren wesentlich weniger Dampf zum Erwärmen des thermisch nachzubehandelnden Spaltproduktes benötigt wird. Bei einem genügend großen Betrag an frei werdender Reaktionswärme bei der thermischen Nachbehandlung des Spaltproduktes kann auf den Einsatz von Dampf zum Erwärmen des Spaltproduktes völlig verzichtet werden. Im Gegensatz zu Verfahren bzw. Vorrichtungen, bei welchen Dampf oder andere geeignete Wärmeträger dauernd zum Erwärmen des Spaltproduktes eingesetzt wird, tritt der Effekt des Foulings bei Einsatz des erfindungsgemäßen Verfahrens zur Behandlung des Spaltproduktes in wesentlich geringerem Ausmaß bzw. gar nicht auf.

Nachfolgend wird das erfindungsgemäße Verfahren beispielhaft anhand der Nachbehandlung des bei der Spaltung von CHP in Phenol und Aceton entstehenden Spaltproduktes beschrieben, ohne dass das erfindungsgemäße Verfahren auf diese Ausführungsart beschränkt sein soll.

Das erfindungsgemäße Verfahren zur thermischen Nachbehandlung von Spaltprodukt, das bei der säurekatalysierten Spaltung von Cumolhydroperoxid in Phenol und Aceton anfällt, hat den Zweck, den Anteil an Dimethylphenylcarbinol (DMPC) und Dicumylperoxid (DCP) im Spaltprodukt zu verringern, da diese Verbindungen bei der anschließenden Aufarbeitung des Spaltproduktes, bei welcher mehrere destillative Schritte zur Stofftrennung durchgeführt werden, mit anderen Verbindungen oder mit sich selbst zu hochsiedenden, Teerähnlichen Verbindungen weiterreagieren. Diese hochsiedenden Verbindungen können störend in den weiteren Prozessschritten zur Aufarbeitung von Spaltprodukt wirken. Durch die Bildung der Hochsieder wird außerdem die Ausbeute im Gesamtprozess der Hock'schen Phenolsynthese deutlich verringert.

Durch die erfindungsgemäße, thermische Behandlung bzw. Nachbehandlung von Spaltprodukt werden das in diesem enthaltende DMPC in α-Methylstyrol (AMS) und Wasser und das ebenfalls vorhandene DCP in Phenol, AMS und Aceton gespalten. Das bei diesen Reaktionen entstehende AMS lässt sich bei der weiteren Aufarbeitung des Spaltproduktes von diesem abtrennen und zu Cumol hydrieren, welches als Ausgangsstoff in den Gesamtprozess der Phenolherstellung rückgeführt werden kann. Auf diese Weise werden die Ausbeuteverluste durch Bildung von Nebenprodukten verringert.

Zur Durchführung der oben genannten Reaktionen muss das Spaltprodukt auf eine bestimmte Temperatur erwärmt werden. Es wurde gefunden, dass bei Temperaturen über 110 °C auch bei noch nicht vollständigem Umsatz an DCP die Umwandlung von DMPC in AMS und Wasser bereits vollständig ist. Somit muss zur Einstellung optimaler Bedingungen bei Fahrweisen oberhalb 110°C allein der DCP-Restgehalt nach der thermischen Nachbehandlung überprüft werden. Vorzugsweise beträgt der Restgehalt an DCP im erfindungsgemäß thermisch nachbehandelten Spaltprodukt von 0,01 bis 0,05 Gew.-%, vorzugsweise von 0,01 bis 0,02 Gew.-%. Höhere Werte führen zu einer Verschlechterung der Selektivität des Gesamtprozesses eben über diese DCP-Verluste, die darüber hinaus zu höheren Gehalten an o-Kresol im Reinphenol führen können, niedrige Werte von unter 0,01 Gew.-% führen zu einer zu hohen Nebenproduktbildung von Hochsiedern aus AMS in der thermischen Nachbehandlung. Üblicherweise wird der DCP-Restgehalt analytisch ermittelt.

Aus oben genannten Gründen wird das thermisch nachzubehandelnde Spaltprodukt auf eine Temperatur von über 100 °C, vorzugsweise von über 115 °C erwärmt. Diese thermische Nachbehandlung wird auch als Temperung bezeichnet.

Zur Nachbehandlung des Spaltproduktes wird dieses in einen Reaktor, vorzugsweise in einen Rohrreaktor überführt und erwärmt. Erfindungsgemäß erfolgt das Erwärmen bzw. Erhitzen des Spaltproduktgemischs durch Nutzen der Reaktionswärme, die beim Ablauf zumindest einer exothermen Reaktion im Spaltprodukt entsteht. Erfindungsgemäß ist eine der exothermen Reaktionen die säurekatalysierte Spaltung von CHP. Da das Erwärmen des Spaltproduktes durch Nutzung der Reaktionswärme einer exothermen Reaktion direkt erfolgt, kann auf eine indirekte Wärmeübertragung mittels Wärmeübertragern zur Erwärmung des Spaltproduktes unter Umständen ganz verzichtet werden.

Auch durch die Spaltung von DMPC in AMS und Wasser, vor allem aber auch durch die Spaltung von DCP in Phenol, Aceton und AMS wird, da es sich ebenfalls um exotherme Reaktionen handelt, ebenfalls Reaktionswärme frei, die einer definierten Erhöhung der Temperatur im Spaltprodukt entspricht. Diese Temperaturdifferenz liegt je nach Anfangsgehalten an DMPC und DCP üblicherweise zwischen 10 und 20 °C. Typische Konzentrationen an DMPC sind Konzentrationen von 0,5 bis 2 Gew.-%. Typische Konzentrationen von DCP liegen im Bereich von 2 bis 6 Gew.-%. Das erfindungsgemäße Verfahren soll aber nicht auf die angegebenen Konzentrationen für DCP oder DMPC beschränkt sein.

Die durch diese oben genannten exothermen Reaktionen frei werdende Wärmemenge muss bei der Berechnung der erforderlichen Anfangskonzentration von CHP im Spaltprodukt vor der thermischen Nachbehandlung, die zum Erwärmen des Spaltproduktes auf die gewünschte Temperatur notwendig ist, berücksichtigt werden.

Als Anhaltspunkt zur Berechnung der notwendigen CHP-Anfangskonzentration kann die Faustformel dienen, dass die Spaltung von einer 1 Gew.-%igen CHP-Lösung ungefähr so viel Wärme freisetzt, wie zur Erhöhung der Temperatur der Lösung um 6,8 bis 7,0 °C notwendig ist. Eine 6 Gew.-%ige CHP-Lösung würde also durch Spaltung des gesamten CHPs um 40,8 bis 42 °C erwärmt werden. Die Faustformel gilt für die bei der CHP-Spaltung üblicherweise eingesetzten Lösungen. Diese weisen üblicherweise zumindest Cumol, Phenol und Aceton aber nur geringe Mengen (von 0 bis 15 Gew.-%) an Wasser auf. Auf Grund der höheren Wärmekapazität des Wassers würde die Spaltung von CHP in einer Lösung bzw. Dispersion, die 99 Gew.-% Wasser und 1 Gew.-% CHP aufweist diese Lösung nur um 3,5 °C erhöhen. Für Spaltgemische, die einen höheren Wasseranteil als üblich aufweisen, muss deshalb der Erwärmungsfaktor neu bestimmt werden. Diese Bestimmung kann auf eine dem Fachmann bekannte Weise in einfachen Vorversuchen erfolgen.

Erfindungsgemäß wird das zur Erzeugung der Wärme notwendige zusätzliche CHP nachträglich in das Spaltprodukt zugegeben, sofern es im Spaltproduktgemisch noch nicht ausreichend vorhanden ist.

Vorzugsweise wird zur Spaltung von CHP Schwefelsäure als Katalysator eingesetzt. Vorzugsweise weist das Spaltproduktgemisch eine Konzentration an Schwefelsäure von 50 bis 1000 wppm auf. Es kann vorteilhaft sein, die Säureaktivität, dass heißt die Säurestärke des Spaltproduktes vor der thermischen Behandlung zu verändern. Die Säurestärke ist abhängig von der Säurekonzentration und der Konzentration an Wasser im Spaltgemisch. Je höher der Wassergehalt im Spaltgemisch ist, desto mehr Säure muss dem Spaltgemisch zudosiert werden, um auf die gleiche Säureaktivität zu kommen, wobei die Wasserkonzentration bei der Berechnung der Säurestärke zum Quadrat eingeht. So beträgt zum Beispiel die Säurestärke einer Spaltgemisch-Lösung, die 200 wppm Schwefelsäure und 2 Gew.-% Wasser nur ein Sechzehntel der Säurestärke einer Spaltgemisch-Lösung die 200 wppm Schwefelsäure und 0,5 Gew.-% Wasser aufweist.

Die ideale Säurestärke und damit die ideale Zusammensetzung des Spaltgemisches in Bezug auf Säurekonzentration und Wasserkonzentration kann durch einfache Vorversuche ermittelt werden. Bei Spaltgemischen, die eine Wasserkonzentration von bis zu 6 Gew.-% aufweisen, erweist sich eine Schwefelsäurekonzentration von 100 bis 500 wppm im Spaltgemisch als besonders vorteilhaft. Zur Erhöhung der Säurestärke wird üblicherweise Schwefelsäure nachdosiert. Zur Senkung der Säurestärke kann dem Spaltprodukt eine Base, wie z.B. Phenolatlauge, Ammoniak oder Natronlauge, oder Wasser hinzugefügt werden. Vorzugsweise wird dem Spaltprodukt Wasser hinzugefügt.

In einer besonders bevorzugten Ausführungsart des erfindungsgemäßen Verfahrens weist das thermisch zu behandelnde Spaltprodukt eine CHP-Konzentration auf, die in Kombination mit den Konzentrationen an weiteren Verbindungen, die exotherm reagieren, bei der Spaltreaktion eine genau so große Wärmemenge freisetzt, dass das Spaltproduktgemisch auf die gewünschte Temperatur für die thermische Nachbehandlung aufgeheizt wird.

Bei dieser Ausführungsart des erfindungsgemäßen Verfahrens wird einem Spaltgemisch vor der Spaltung so viel CHP zugeführt, dass die Konzentration an CHP größer ist als die Konzentration, die zur Erwärmung bzw. zur thermischen Nachbehandlung des Spaltproduktgemisches nötig wäre. Dieses Spaltgemisch wird auf übliche Weise gespalten, wobei das Spaltgemisch durch Kühlen in einem Temperaturbereich von 40 bis 85 °C, vorzugsweise von 45 bis 75 °C, gehalten wird. Erst wenn das Spaltprodukt die gewünschte CHP-Konzentration aufweist und somit das Spaltproduktgemisch durch die zumindest eine ablaufende exotherme Reaktion auf die gewünschte Temperatur erwärmt werden kann, wird bei einem diskontinuierlichen Betrieb die Kühlung abgestellt, bzw. wird das Spaltproduktgemisch bei einer kontinuierlichen Fahrweise in einen Reaktor oder Reaktorbereich zur thermischen Nachbehandlung überführt, in welchem keine Kühlung stattfindet. Die notwendigen Verweilzeiten und damit die CHP-Konzentration können durch einfache Vorversuche ermittelt werden.

Es kann vorteilhaft sein, das erfindungsgemäße Verfahren in einem für dieses Verfahren besonders geeigneten Reaktor, wie er unten beschrieben wird durchzuführen. Besonders vorteilhaft kann es sein, die Spaltung von CHP und die thermische Nachbehandlung des Spaltproduktes in einem Reaktor durchzuführen. Es ist aber ebenfalls möglich, das erfindungsgemäße Verfahren in einer Vorrichtung, wie sie im Stand der Technik zur Durchführung von Spaltung und thermischer Nachbehandlung beschrieben wird, durchzuführen.

In einer weiteren besonders bevorzugten Ausführungsart des erfindungsgemäßen Verfahrens wird zum Spaltprodukt, welches eine CHP-Konzentration aufweist die nicht ausreichend ist, das Spaltprodukt zur thermischen Nachbehandlung genügend zu erwärmen, zusätzlich CHP zudosiert.

Vorzugsweise wird das CHP als Konzentrat, welches von 65 bis 90 Gew.-% CHP aufweist zudosiert. Das Zudosieren erfolgt vorzugsweise so, dass eine ausreichende Durchmischung des zudosierten CHPs mit dem Spaltprodukt erreicht wird. Dies kann auf eine dem Fachmann bekannte Weise, z.B. durch Einbauten, die eine vollständige Durchmischung ermöglichen wie z.B. statische Mischer, sichergestellt werden. Vorzugsweise erfolgt die Zudosierung des CHPs auf der Saugseite der Pumpe, welche das zu behandelnde Spaltprodukt in den Rohrreaktor pumpt. Auch auf diese Weise wird das Erreichen einer vollständigen Durchmischung des Spaltproduktes mit dem zudosierten CHP sichergestellt. Eine ausreichende Durchmischung des CHPs mit dem thermisch zu behandelnden Spaltprodukt ist notwendig, um eine lokale Überhitzung des Spaltproduktes bei der Temperung zu vermeiden.

Die bei beiden Ausführungsarten des erfindungsgemäßen Verfahrens notwendige Konzentration an CHP im Spaltprodukt beträgt, abhängig von der Starttemperatur des Spaltproduktes und von der DCP-Ausgangskonzentration, von 5 bis 10 Gew.-%. Zur Berechnung der notwendigen CHP-Konzentration kann die oben genannte Faustformel herangezogen werden. So beträgt z.B. bei einer Starttemperatur des Spaltproduktes von 40 °C und einem DCP-Gehalt von 4 Gew.-% die erforderliche CHP-Konzentration vor Eintritt in die thermische Nachbehandlung ca. 8,5 Gew.-% um auf eine Endtemperatur von 115 °C zu kommen. Die Aufheizzeit bis 100 °C beträgt dabei üblicherweise weniger als 30 sec. In der anschließenden eigentlichen Temperung steigt die Temperatur des Gemisches im Verweilzeitreaktor auf eine Temperatur von ca. 115 °C an. Die Verweilzeit des Spaltproduktgemisches im Verweilzeitreaktor ist abhängig von der Säurestärke. Je nach Säurestärke beträgt die Verweilzeit üblicherweise von 30 bis 300 sec.

Nach der thermischen Behandlung des Spaltproduktes im Reaktor kann das behandelte Spaltprodukt in einem Kühler auf eine Endtemperatur von üblicherweise 40 bis 70 °C gebracht werden. Das erfindungsgemäß behandelte Spaltprodukt wird einer Weiterbehandlung oder Aufarbeitung zugeführt. Üblicherweise wird das thermisch behandelte Spaltprodukt so aufgearbeitet, dass Aceton und Phenol destillativ voneinander und von weiteren im thermisch behandelten Spaltprodukt vorhandenen Verbindungen getrennt werden. Die Aufarbeitung dieser Spaltproduktströme ist dem Fachmann bekannt.

Bei allen Ausführungsarten des erfindungsgemäßen Verfahrens kann es vorteilhaft sein, dem Spaltprodukt vor der thermischen Behandlung Wasser zuzusetzen. Besonders bevorzugt wird dem Spaltprodukt vor der thermischen Behandlung so viel Wasser zugesetzt, dass die Konzentration von Wasser im Spaltprodukt von 0,5 bis 3,0 Gew.-%, vorzugsweise von 1,5 bis 2 Gew.-% und ganz besonders bevorzugt von 1,8 Gew.-% beträgt.

Das erfindungsgemäß thermisch behandelte Spaltprodukt weist eine Konzentration an DCP von 0,01 bis 0,05, vorzugsweise von 0,01 bis 0,02 Gew.-% und eine Konzentration an DMPC von 0,05 bis 0,2 Gew.-% auf. CHP ist im thermisch behandelten Spaltprodukt nicht mehr nachweisbar.

Das erfindungsgemäße Verfahren kann bei allen Verfahren eingesetzt werden, in denen Alkylarylhydroperoxide gespalten werden. Alkylarylhydroperoxide können z.B. Cumolhydroperoxid, sek-Butylbenzolhydroperoxid aber auch substituierte Alkylbenzolhydroperoxide oder Alkylhydroperoxide anderer Aromaten, wie z. B. Naphtahlin, sein. Vorzugsweise wird das erfindungsgemäße Verfahren bei der Nachbehandlung von Spaltprodukt aus der Spaltung von Alkylarylhydroperoxiden eingesetzt, bei denen die Spaltung eine exotherme Reaktion ist. Es ist aber auch möglich das erfindungsgemäße Verfahren zur Nachbehandlung von Spaltprodukt, welches durch die Spaltung von mehr als einem Alkylarylhydroperoxid erhalten wird, einzusetzen. In diesem Fall muss zumindest eine der Spaltreaktionen eine exotherme Reaktion sein. Ganz besonders bevorzugt wird das erfindungsgemäße Verfahren zur Nachbehandlung des bei der säurekatalysierten Spaltung von CHP in Phenol und Aceton entstehenden Spaltproduktes bzw. zur Spaltung von CHP mit kombinierter thermischer Nachbehandlung des Spaltproduktes eingesetzt.

Das erfindungsgemäße Verfahren kann kontinuierlich oder diskontinuierlich durchgeführt werden. Vorzugsweise wird das erfindungsgemäße Verfahren kontinuierlich durchgeführt.

Das erfindungsgemäße Verfahren kann sowohl zur Nachbehandlung von Spaltprodukt, welches bei der Spaltung in heterogener Phase anfällt, als auch zur Nachbehandlung von Spaltprodukt, welches bei der Spaltung in homogener Phase anfällt, verwendet werden.

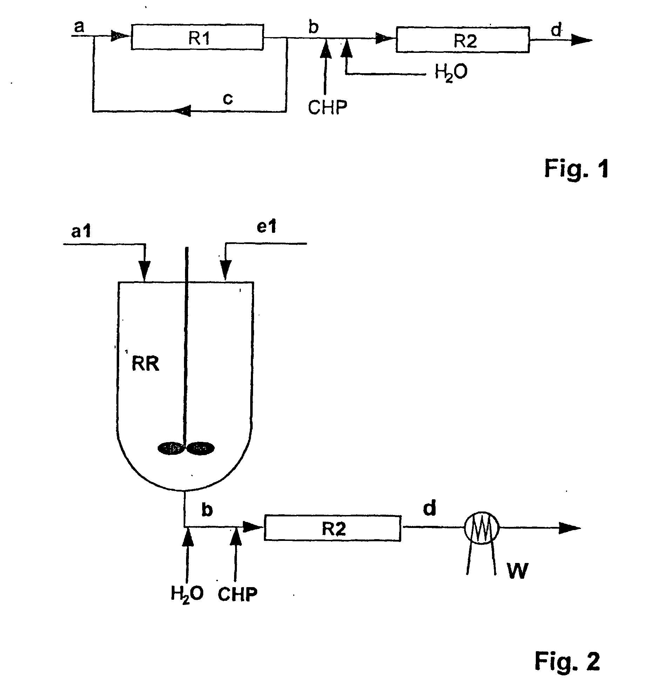

Es kann vorteilhaft sein, das erfindungsgemäße Verfahren in einem für dieses Verfahren besonders geeigneten Reaktor durchzuführen. Dabei wird die säurekatalysierte Spaltung von CHP und die thermische Nachbehandlung des Spaltproduktes in einem Reaktor durchgeführt. Es ist aber auch möglich das erfindungsgemäße Verfahren in bestehenden Anlagen zur Spaltung von CHP, welche über zumindest einen Spaltreaktor und zumindest einen weiteren Reaktor zur thermischen Nachbehandlung des Spaltproduktes verfügen, wie z.B. in den Abbildungen Fig. 1 und Fig.2 dargestellt, einzusetzen.

Vorzugsweise wird ein erfindungsgemäßer Reaktor zur Herstellung von Phenol und Aceton durch säurekatalysierte Spaltung von Cumolhydroperoxid, welcher dadurch gekennzeichnet ist, dass er zumindest zwei Bereiche aufweist, von denen zumindest ein Bereich mit einer Vorrichtung zum Abführen von Wärme ausgerüstet ist und zumindest ein weiterer Bereich Strömungsrohrcharakteristik aufweist, eingesetzt.

In einer bevorzugten Ausführungsform wird in einem Bereich des Reaktors die Spaltung von Cumolhydroperoxid durchgeführt und in einem anderen Bereich des Reaktors die Nachbehandlung des Spaltproduktes durchgeführt. In einer weiteren bevorzugten Ausführungsform weist der Reaktor eine Rückführung auf, mit der zumindest ein Teil des Produktes aus einem Bereich, der eine Vorrichtung zum Abführen von Wärmeenergie aufweist, in den Zulauf des Reaktors zurückgeführt werden kann. Vorzugsweise weist der Reaktor zumindest eine Rückführung auf, mit der zumindest ein Teil des Produktes aus einem Bereich der Strömungsrohrcharakteristik aufweist, in den Zulauf des Reaktors und/oder in einen Bereich, der Strömungsrohrcharakteristik aufweist, zurückgeführt werden kann. Weiterhin kann der Reaktor zumindest eine Zudosiervorrichtung zwischen einem Bereich des Reaktors, der eine Vorrichtung zum Abführen von Wärmeenergie aufweist, und einem Bereich des Reaktors, der Strömungsrohrcharakteristik aufweist, aufweisen.

Bei diesem erfindungsgemäßen Reaktor sind der Spaltreaktor und der zur thermischen Nachbehandlung notwendige Reaktor kombiniert. Dies wird dadurch erreicht, dass ein Reaktor verwendet wird, in welchem ein Temperaturprofil eingestellt werden kann. Vorzugsweise weist ein solcher erfindungsgemäßer Reaktor ein Temperaturprofil der Gestalt auf, dass in dem Bereich des kombinierten Reaktors, in welchem die Spaltung stattfinden soll, eine Temperatur, die für die Spaltung von CHP bevorzugt verwendet wird, wie z.B. eine Temperatur von 40 bis 85°C, eingestellt werden kann. Dies kann z.B. dadurch erreicht werden, dass in zumindest einem Bereich des Reaktors Wärmeübertragungsvorrichtungen, wie z.B. Wärmeübertrager, vorhanden sind, mit welchen das zu spaltende Gemisch auf der gewünschten Temperatur, vorzugsweise durch Abführen von Reaktionswärme, gehalten werden kann. Eine mögliche Ausführungsform ist z.B. die Hintereinanderschaltung von mehreren Wärmeübertragern. In diesem Bereich findet vorzugsweise die Spaltung von CHP statt. Des Weiteren ist in einem solchen erfindungsgemäßen Reaktor zumindest ein Bereich, in welchem die thermische Nachbehandlung stattfinden soll, vorhanden, welcher vorzugsweise eine Strömungsrohrcharakteristik aufweist. Dieser Bereich des kombinierten Reaktors weist vorzugsweise keine Vorrichtung zum Erwärmen des zu behandelnden Spaltproduktes auf. Es kann vorteilhaft sein, wenn dieser Bereich des Reaktors eine Vorrichtung zum Kühlen des zu behandelnden Spaltproduktgemisches aufweist. Bei genügend vorsichtiger Fahrweise des erfindungsgemäßen Verfahrens kann aber auch auf eine solche Kühlung verzichtet werden.

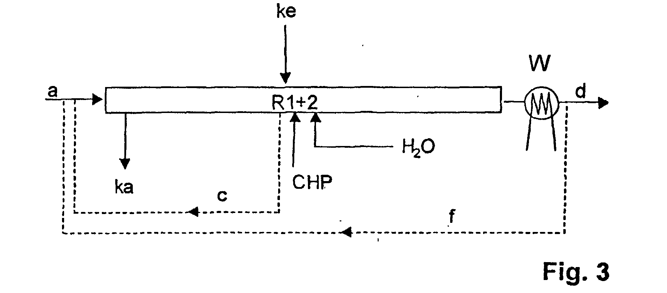

Je nach Ausführungsart des erfindungsgemäßen Verfahrens kann es vorteilhaft sein, den erfindungsgemäßen Reaktor zur Spaltung von CHP und Nachbehandlung des bei der Spaltung entstandenen Spaltproduktes mit zumindest einem, vorzugsweise mit zumindest zwei Zudosiereinrichtungen zu versehen, mit denen es möglich ist, Wasser und/oder CHP, bzw. ein CHP aufweisendes Gemisch, in den Bereich des erfindungsgemäßen Reaktors einzudosieren, in welchem die thermische Nachbehandlung des Spaltproduktes stattfinden soll. Eine mögliche Ausführungsart eines solchen erfindungsgemäßen Reaktors ist beispielsweise in Abbildung Fig.3 dargestellt.

Der Bereich der thermischen Nachbehandlung im kombinierten Reaktor ist vorzugsweise so gestaltet, dass die im erfindungsgemäßen Verfahren genannten Parameter, wie z.B. Temperatur, Aufheizzeit und Verweilzeit, eingehalten werden.

Es kann vorteilhaft sein, wenn der erfindungsgemäß kombinierte Reaktor mit zumindest einer Möglichkeit ausgestattet ist, zumindest einen Teil des gespaltenen Spaltproduktes und/oder zumindest einen Teil des nachbehandelten Spaltproduktes in den Reaktor zurückzufahren. Die Rückführung kann so ausgeführt sein, dass ein Teil des Spaltproduktes vor dem Eintritt in den Bereich des Reaktors, in welchem die Nachbehandlung stattfindet, abgezweigt werden kann und in den Zulauf zum Reaktor zurückgeführt werden kann. Die Rückführung kann aber auch so ausgeführt sein, dass ein Teil des thermisch nachbehandelten Spaltproduktgemisches in den Zulauf zum Reaktor oder in den Bereich des Reaktors zurückgeführt werden kann, in welchem die thermische Nachbehandlung des Spaltproduktes einsetzt. Es kann ebenso vorteilhaft sein, die genannten Rückführungen kombiniert vorzusehen.

Das erfindungsgemäße Verfahren bzw. die erfindungsgemäße Vorrichtung wird in den Abbildungen Fig. 1 bis Fig. 3 beispielhaft beschrieben, ohne dass das Verfahren bzw. die Vorrichtung auf diese Ausführungsarten beschränkt sein soll.

In Fig. 1 ist schematisch ein Verfahren zur Spaltung von CHP dargestellt. Über Leitung **a** wird ein Gemisch, welches das zu spaltende **CHP** aufweist in einen ersten Reaktor, den Spaltreaktor, eingespeist. Bei dem Spaltreaktor **R1** muss es sich nicht um nur einen Reaktor handeln, der z.B. als Rohrreaktor mit Rückführung oder als rückvermischter Apparat ausgeführt sein kann, es können auch mehrere hintereinander geschaltete Reaktoren als Spaltreaktor bezeichnet werden. Das aus dem Spaltreaktor austretende Spaltprodukt wird im Falle eines Rohrreaktors zumindest teilweise über Leitung **c** im Kreis zurück in den Spaltreaktor gefahren. Über Leitung **b** wird ein Teil des Spaltproduktgemisches in einen zweiten Reaktor **R2** gefahren, in welchem die thermische Nachbehandlung stattfindet. Vor dem Reaktor **R2** kann über zwei Leitungen zusätzlich Cumolhydroperoxid **(CHP)** und/oder Wasser **(H**_{**2**}**O)** in das Spaltproduktgemisch gefahren werden. Über Leitung **d** verlässt das nachgetemperte Spaltproduktgemisch den Reaktor **R2** und kann einer Aufarbeitung zugeführt werden.

In Fig. 2 ist ebenfalls schematisch die Spaltung von CHP dargestellt. Über Leitung **a1** wird ein Gemisch, welches das zu spaltende CHP aufweist in einen ersten Reaktor, den Spaltreaktor **RR,** bei dem es sich um einen rückvermischten Reaktor handeln kann, eingespeist. Über Leitung **e1** kann der für die säurekatalysierte Spaltung notwendige Katalysator, z.B. Schwefelsäure, in den Spaltreaktor zudosiert werden. Das aus dem Spaltreaktor austretende Spaltprodukt wird über Leitung **b** in einen zweiten Reaktor **R2** gefahren, in welchem die thermische Nachbehandlung stattfindet. Vor dem Reaktor **R2** kann über zwei Leitungen zusätzlich Cumolhydroperoxid **(CHP)** und/oder Wasser **(H**_{**2**}**O)** in das Spaltproduktgemisch gefahren werden. Über Leitung **d,** in der ein Wärmeübertrager **W** vorgesehen ist, mit dem das nachbehandelte Spaltproduktgemisch abgekühlt werden kann, verlässt das nachgetemperte Spaltproduktgemisch den Reaktor **R2** und kann einer Aufarbeitung zugeführt werden.

In Fig. 3 ist schematisch die Spaltung von CHP in einem erfindungsgemäßen Reaktor dargestellt. Über Leitung **a** wird ein Gemisch, welches das zu spaltende CHP aufweist in einen Reaktor **R1+2,** den kombinierten Spalt- und Nachbehandlungsreaktor, eingespeist. Bei diesem Reaktor **R1+2** handelt es sich vorzugsweise um einen Rohrreaktor. Ein Teil des Reaktors **R1+2** kann mittels einer Kühlung auf einer gewünschten Temperatur gehalten werden, indem über **ke** Kühlmittel, z.B. Wasser, in z.B. einen Kühlmantel, eingespeist wird. Das Kühlmittel verlässt den Reaktor über **ka.**

Am Ende des mit einer Kühlung versehenen Abschnitts des Reaktors oder am Anfang des nicht mit einer Kühlung versehenen Abschnitts des Reaktors kann eine Einspeisung für Wasser **H**_{**2**}**O** und/oder eine Einspeisung für Cumolhydroperoxid-Konzentrat **CHP** vorgesehen sein. In dem Abschnitt des Reaktors, der keine Kühlung aufweist, findet die thermische Nachbehandlung des Spaltproduktes statt, welches durch die bei der Spaltung von CHP frei werdende Wärme auf die gewünschte Temperatur gebracht wird. Das aus dem Reaktor austretende, thermisch nachbehandelte Spaltproduktgemisch kann in einem Wärmeübertrager abgekühlt werden und über Leitung **d** einer Aufarbeitung zugeführt werden.

Der erfindungsgemäße Reaktor kann mit einer oder mehreren Möglichkeiten zur Rückführung zumindest eines Teils des nachbehandelten Spaltproduktes und/oder zumindest eines Teils des Spaltproduktes versehen sein. Diese Möglichkeiten sind in Fig. 3 gestrichelt dargestellt. So kann über Leitung **c** zumindest ein Teil des Spaltproduktes in den Reaktor bzw. in die Zuleitung **a** zum Reaktor zurückgefahren werden. Über Leitung **f** kann das nachbehandelte und im Wärmeübertrager **W** abgekühlte Spaltprodukt ebenfalls in den Reaktor bzw. in die Zuleitung a zum Reaktor zurückgefahren werden.

### Beispiel 1:

Ein Spaltprodukt, welches 40 Gew.-% Phenol, 4,0 Gew.-% DCP, 7,8 Gew.-% CHP und 0,8 Gew.-% DMPC aufweist, wurde ausgehend von 50 °C und einer Schwefelsäurekonzentration von 200 wppm durch Spaltung des CHPs und des DCPs innerhalb von 30 sec. auf eine Temperatur von 105 °C aufgeheizt. Bei einer Verweilzeit von 120 sec. im Reaktor vollzog sich die Spaltung von DCP und DMPC bei einer Temperatur von 105 °C bis 120 °C. Das so nachbehandelte Spaltproduktgemisch wurde in einem Wärmetauscher wieder schnell auf eine Temperatur von 45 °C abgekühlt. Der DCP-Restgehalt des thermisch behandelten Spaltproduktes lag bei 0.02 Gew.-%.

### Beispiel 2:

Einem Spaltproduktgemisch, welches 40 Gew.-% Phenol, 3,0 Gew.-% DCP, 0,8 Gew.-% DMPC sowie 2 Gew.-% CHP enthielt und eine Temperatur von 60 °C aufwies, wurde so viel einer 67 %igen CHP-Lösung und so viel Wasser zudosiert, dass das Spaltproduktgemisch 5,8 Gew.-% CHP und 1,8 Gew.-% Wasser aufwies. Die Säurekonzentration betrug 500 wppm. Die Zudosierung von CHP-Lösung und Wasser erfolgte auf der Saugseite der Förderpumpe, die das zu behandelnde Spaltprodukt durch den Reaktor beförderte. Durch die Zugabe des CHPs erhöhte sich durch die exotherme Spaltung des CHPs die Temperatur des Spaltproduktgemisches auf eine Temperatur von 100 °C. Bei dieser Temperatur setzte die DCP und DMPC Spaltung ein, wobei sich im Spaltproduktgemisch eine Temperatur bis 112 °C bei einer Verweilzeit von 230 sec. einstellte. Das so nachbehandelte Spaltproduktgemisch wurde in einem Wärmetauscher wieder schnell auf eine Temperatur von 45 °C abgekühlt. Der DCP-Restgehalt des thermisch behandelten Spaltproduktes lag bei 0.01 Gew.-%.

## Patentansprüche

1. Verfahren zur thermischen Nachbehandlung von Spaltprodukt aus der säurekatalysierten Spaltung von Cumolhydroperoxid in Phenol und Aceton, wobei das thermisch zu behandelnde Spaltprodukt in einem Reaktor erwärmt wird,
**dadurch gekennzeichnet,**
**dass** zum Erwärmen des thermisch zu behandelnden Spaltproduktes im Reaktor die Reaktionswärme zumindest einer in diesem Reaktor ablaufenden exothermen Reaktion genutzt wird.

2. Verfahren nach Anspruch 1,
**dadurch gekennzeichnet,**
**dass** eine der im Reaktor ablaufenden exothermen Reaktionen die Spaltung von Cumolhydroperoxid ist.

3. Verfahren nach zumindest einem der Ansprüche 1 oder 2,
**dadurch gekennzeichnet,**
**dass** das zu behandelnde Spaltprodukt vor der thermischen Nachbehandlung eine Cumolhydroperoxid-Konzentration von 5 bis 10 Gew.-% aufweist.

4. Verfahren nach zumindest einem der Ansprüche 1 bis 3,
**dadurch gekennzeichnet,**
**dass** dem zu behandelnden Spaltprodukt Cumolhydroperoxid zudosiert wird.

5. Verfahren nach zumindest einem der Ansprüche 1 bis 4,
**dadurch gekennzeichnet,**
**dass** dem zu behandelnden Spaltprodukt Wasser zudosiert wird.

6. Verfahren nach zumindest einem der Ansprüche 1 bis 5,
**dadurch gekennzeichnet,**
**dass** das thermisch zu behandelnde Spaltprodukt auf eine Temperatur von über 100 °C erwärmt wird.

7. Verfahren nach Anspruch 6,
**dadurch gekennzeichnet,**
**dass** das thermisch zu behandelnde Spaltprodukt auf eine Temperatur von über 115 °C erwärmt wird.

8. Verfahren nach zumindest einem der Ansprüche 1 bis 7
**dadurch gekennzeichnet,**
**dass** der Restgehalt an Dicumylperoxid in dem thermisch behandelten Spaltprodukt von 0,01 bis 0,05 Gew.-% beträgt.

9. Verfahren nach Anspruch 8,
**dadurch gekennzeichnet,**
**dass** der Restgehal t an Di cumyl peroxid in dem thermisch behandel ten Spaltprodukt von 0,01 bis 0,02 Gew.-% beträgt.

10. Verfahren nach zumindest einem der Ansprüche 1 bis 9,
**dadurch gekennzeichnet,**
**dass** die säurekatalysierte Spaltung von Cumolhydroperoxid und die thermische Nachbehandlung des Spaltproduktes in einem Reaktor durchgeführt werden.

11. Verfahren nach zumindest einem der Ansprüche 1 bis 10,
**dadurch gekennzeichnet,**
**dass** die säurekatalysierte Spaltung von Cumolhydroperoxid und die thermische Nachbehandlung des Spaltproduktes kombiniert durchgeführt werden.

12. Verfahren nach zumindest einem der Ansprüche 1 bis 11,
**dadurch gekennzeichnet,**
**dass** als Reaktor ein Rohrreaktor eingesetzt wird.

13. Verfahren nach zumindest einem der Ansprüche 1 bis 12, zur Herstellung von Phenol.

14. Verfahren nach einem der vorstehenden Ansprüche,
**dadurch gekennzeichnet,**
**dass** das Verfahren in einem Reaktor durchgeführt wird, der zumindest zwei Bereiche aufweist, von denen zumindest ein Bereich mit einer Vorrichtung zum Abführen von Wärme ausgerüstet ist und zumindest ein weiterer Bereich Strömungsrohrcharakteristik aufweist.

15. Verfahren nach Anspruch 14,
**dadurch gekennzeichnet,**
**dass** in einem Bereich des Reaktors die Spaltung von Cumolhydroperoxid durchgeführt wird und in einem anderen Bereich des Reaktors die Nachbehandlung des Spaltproduktes durchgeführt wird.

16. Verfahren nach zumindest einem der Ansprüche 14 oder 15,
**dadurch gekennzeichnet,**
**dass** der Reaktor eine Rückführung aufweist, mit der zumindest ein Teil des Produktes aus einem Bereich, der eine Vorrichtung zum Abführen von Wärmeenergie aufweist, in den Zulauf des Reaktors zurückgeführt werden kann.

17. Verfahren nach zumindest einem der Ansprüche 14 bis 16,
**dadurch gekennzeichnet,**
**dass** der Reaktor zumindest eine Rückführung aufweist, mit der zumindest ein Teil des Produktes aus einem Bereich, der Strömungsrohrcharakteristik aufweist, in den Zulauf des Reaktors und/oder in einen Bereich, der Strömungsrohrcharakteristik aufweist, zurückgeführt werden kann.

18. Verfahren nach zumindest einem der Ansprüche 14 bis 17,
**dadurch gekennzeichnet,**
**dass** der Reaktor zumindest eine Zudosiervorrichtung zwischen einem Bereich des Reaktors, der eine Vorrichtung zum Abführen von Wärmeenergie aufweist, und einem Bereich des Reaktors, der Strömungsrohrcharakteristik aufweist, aufweist.

## Claims

1. A process for the thermal after-treatment of cleavage product from the acid-catalyzed cleavage of cumene hydroperoxide into phenol and acetone, whereby the cleavage product to be treated thermally is heated in a reactor, **characterized in that** for heating the cleavage product to be treated thermally in a reactor, the heat generated by at least one exothermic reaction which occurs in the reactor is used.

2. The process as claimed in claim 1, **characterized in that** one of the exothermic reactions occurring in the reactor is the cleavage of cumene hydroperoxide.

3. The process as claimed in at least one of claims 1 or 2, **characterized in that** the cleavage product to be treated has a cumene hydroperoxide concentration ranging from 5 to 10 percent by weight before the thermal after-treatment.

4. The process as claimed in at least one of claims 1 to 3, **characterized in that** cumene hydroperoxide is added to the cleavage product to be treated.

5. The process as claimed in at least one of claims 1 to 4, **characterized in that** water is added to the cleavage product to be treated.

6. The process as claimed in at least one of claims 1 to 5, **characterized in that** the cleavage product to be treated thermally is heated to a temperature above 100°C.

7. The process as claimed in claim 6, **characterized in that** the cleavage product to be treated thermally is heated to a temperature above 115°C.

8. The process as claimed in at least one of claims 1 to 7, **characterized in that** the residual dicumyl peroxide content of the thermally treated cleavage product ranges from 0.01 to 0.05 percent by weight.

9. The process as claimed in claim 8, **characterized in that** the residual dicumyl peroxide content of the thermally treated cleavage product ranges from 0.01 to 0.02 percent by weight.

10. The process as claimed in at least one of claims 1 to 9, **characterized in that** the acid-catalyzed cleavage of cumene hydroperoxide and the thermal after-treatment of the cleavage product are conducted in one reactor.

11. The process as claimed in at least one of claims 1 to 10, **characterized in that** the acid-catalyzed cleavage of cumene hydroperoxide and the thermal after-treatment of the cleavage product are conducted in combination.

12. The process as claimed in at least one of claims 1 to 11, **characterized in that** the reactor is a tube reactor.

13. The process as claimed in at least one of claims 1 to 12, for preparing phenol.

14. The process as claimed in any of the above claims, **characterized in that** it is conducted in a reactor having at least two regions of which at least one region is equipped with a means for removing heat and at least one other region has plug flow characteristics.

15. The process as claimed in claim 14, **characterized in that** the cleavage of cumene hydroperoxide is conducted in one region of the reactor and the after-treatment of the cleavage product is conducted in another region of the reactor.

16. The process as claimed in at least one of claims 14 or 15, **characterized in that** the reactor has a reci rcul ati on by means of which at least part of the product can be recirculated from a region having a means for removal of heat energy to the inlet of the reactor.

17. The process as claimed in at least one of claims 14 to 16, **characterized in that** the reactor has at least one recirculation by means of which at least part of the product can be recirculated from a region having plug flow characteristics to the inlet of the reactor and/or into a region having plug flow characteristics.

18. The process as claimed in at least one of claims 14 to 17, **characterized in that** the reactor has at least one metering-in facility between a region of the reactor having a means for the removal of heat energy and a region of the reactor having plug flow characteristics.

## Revendications

1. Procédé pour le posttraitement thermique du produit de scission de la scission par catalyse acide de l'hydroperoxyde de cumène dans du phénol et de l'acétone, dans lequel le produit de scission à traiter par voie thermique est chauffé dans un réacteur, **caractérisé en ce que**, pour le chauffage du produit de scission à traiter par voie thermique dans le réacteur, on utilise la chaleur de réaction d'au moins une réaction exothermique se déroulant dans ce réacteur.

2. Procédé selon la revendication 1, **caractérisé en ce que** l'une des réactions exothermiques se déroulant dans le réacteur est la scission d'hydroperoxyde de cumène.

3. Procédé selon l'une au moins des revendications 1 ou 2, **caractérisé en ce que** le produit de scission à traiter présente, avant le posttraitement thermique, une concentration d'hydroperoxyde de cumène de 5 à 10% en poids.

4. Procédé selon l'une au moins des revendications 1 à 3, **caractérisé en ce que** l'on ajoute de l'hydroperoxyde de cumène au produit de scission à traiter.

5. Procédé selon l'une au moins des revendications 1 à 4, **caractérisé en ce que** l'on ajoute de l'eau au produit de scission à traiter.

6. Procédé selon l'une au moins des revendications 1 à 5, **caractérisé en ce que** le produit de scission à traiter est chauffé à une température de plus de 100°C.

7. Procédé selon la revendication 6, **caractérisé en ce que** le produit de scission à traiter par voie thermique est chauffé à une température de plus de 115°C.

8. Procédé selon l'une au moins des revendications 1 à 7, **caractérisé en ce que** la teneur résiduelle en peroxyde de dicumyle dans le produit de scission traité par voie thermique est de 0,01 à 0,05% en poids.

9. Procédé selon la revendication 8, **caractérisé en ce que** la teneur résiduelle en peroxyde de dicumyle dans le produit de scission traité par voie thermique est de 0,01 à 0,02% en poids.

10. Procédé selon l'une au moins des revendications 1 à 9, **caractérisé en ce que** la scission par catalyse acide d'hydroperoxyde de cumène et le posttraitement thermique du produit de scission sont entrepris dans un réacteur.

11. Procédé selon l'une au moins des revendications 1 à 10, **caractérisé en ce que** la scission par catalyse acide d'hydroperoxyde de cumène et le posttraitement thermique du produit de scission sont entrepris de manière combinée.

12. Procédé selon l'une au moins des revendications 1 à 11, **caractérisé en ce que** l'on utilise comme réacteur un réacteur tubulaire.

13. Procédé selon l'une au moins des revendications 1 à 12 pour la préparation de phénol.

14. Procédé selon l'une quelconque des revendications qui précèdent, **caractérisé en ce que** le procédé est entrepris dans un réacteur présentant au moins deux zones, dont au moins une zone est équipée d'un dispositif de dissipation de la chaleur et au moins une autre zone présente une caractéristique de tube à écoulement.

15. Procédé selon la revendication 14, **caractérisé en ce que** l'on entreprend la scission d'hydroperoxyde de cumène dans une zone du réacteur et le posttraitement du produit de scission dans une autre zone du réacteur.

16. Procédé selon l'une au moins des revendications 14 ou 15, **caractérisé en ce que** le réacteur présente un recyclage qui permet de recycler dans l'entrée du réacteur au moins une partie du produit provenant d'une zone présentant un dispositif pour la dissipation de la chaleur.

17. Procédé selon l'une au moins des revendications 14 à 16, **caractérisé en ce que** le réacteur présente au moins un recyclage permettant de recycler au moins une partie du produit provenant d'une zone présentant une caractéristique de tube à écoulement, dans l'entrée du réacteur et/ou dans une zone présentant une caractéristique de tube à écoulement.

18. Procédé selon l'une au moins des revendications 14 à 17, **caractérisé en ce que** le réacteur présente au moins une zone d'addition dosée, entre une zone du réacteur présentant un dispositif pour la dissipation de la chaleur et une zone du réacteur présentant une caractéristique de tube à écoulement.
